**Europäisches Patentamt**

**European Patent Office**

**Office européen des brevets**

(19)

(11) Publication number: **0 356 007**
**A2**

(12) # EUROPEAN PATENT APPLICATION

(21) Application number: **89307516.8**

(22) Date of filing: **24.07.89**

(51) Int. Cl.⁴: **C07K 7/00 , A61K 37/00 , A61K 39/00 , G01N 33/53**

(30) Priority: **22.07.88 GB 8817557**
**02.08.88 GB 8818340**

(43) Date of publication of application:
**28.02.90 Bulletin 90/09**

(84) Designated Contracting States:
**DE FR GB IT**

(71) Applicant: **MEDICAL RESEARCH COUNCIL**
**20 Park Crescent**
**London W1N 4AL(GB)**

(72) Inventor: **Zvelebil, Marketa Juditha Johana Milena**
**52 Great Russell Street Helen Graham House Flat G10 London, WC1B 3BD(GB)**
Inventor: **Cookson, John**
**9 Overstone Road**
**Harpenden Hertfordshire AL5 5PN(GB)**
Inventor: **Sternberg, Michael Joseph Ezra**
**5 Canons Drive**
**Edgware Middlesex HA8 7QX(GB)**
Inventor: **Coates, Anthony Robert Milnes**
**135 Gloucester Road**
**London, S.W.7(GB)**
Inventor: **Mascagni, Paolo**
**6A Free Grove Road**
**London, N7 9JP(GB)**
Inventor: **Gibbons, William Anthony**
**4 Milverton Street**
**London, SE11 4AP(GB)**

(74) Representative: **Eyles, Christopher Thomas**
**W.P. THOMPSON & CO. 50 Lincoln's Inn Fields**
**London WC2A 3PF(GB)**

(54) **Antigenic determinants.**

(57) A synthetic polypeptide is described containing 94 or 104 amino acid residues which, apart from substitution of alanine for cysteine in two places, corresponds to a portion of the natural gag polypeptide sequence of a virus of the HIV-1 type. A number of T- and B-cell epitopes have been located in this polypeptide.

p55

p17    p24    p15
104-mer

280      290      300      310      320
INKIVRMYSPTSILDIRQGPKEPFRDYVDRFYKTLRAEQASQEVKNWMTETL

330      340      350      360      370
LVQNANPDAKTILKALGPAATLEEMMTAAQGVGGPGHKARVLAEAMSQVTNS

*Fig 2.*

## ANTIGENIC DETERMINANTS

This invention relates to antigenic determinants. In particular it relates to synthetic polypeptide sequences which are related to proteins present in the human immunodeficiency virus type-1 (HIV-1).

There is no effective vaccine for the prevention of AIDS (Acquired Immune Deficiency Syndrome). In designing a vaccine for this disease, it is important to consider epitope sequence variation between strains of the human immunodeficiency virus type-1 (HIV-1) because a successful vaccine should be effective against a range of isolates.

The HIV-1 virus contains three major proteins and some smaller proteins, probably about ten in total. The virus is an RNA virus, its genes being made from polyribonucleic acid (RNA) which are reverse transcribed into deoxyribonucleic acid (DNA) on which corresponding precursor proteins are synthesised in reproduction of the virus. These precursor proteins are then cleaved to give the various proteins found in the HIV-1 virus. The other major strain of AIDS virus, which is known as HIV-2, is similar. Also similar is a virus SIV$_{MAC}$ which causes an AIDS-like disease in rhesus macaque monkeys.

Each of the three viruses, i.e. HIV-1, HIV-2, and SIV$_{MAC}$, contain three main genes (5′ to 3′) which code for the proteins gag, pol and env. The proteins p17, p24 (the major capsid protein) and p15 (a nucleic acid binding protein) are encoded by gag; pol specifies a protease, a reverse transcriptase and an endonuclease; whilst env determines the envelope glycoprotein and a transmembrane protein.

A partial gene map of the HIV-1 virus is set out in Table 1 below:

## TABLE 1

| Gene: | LTR | gag | pol | R | sor | .... |
|---|---|---|---|---|---|---|
| Precursor Protein : | . | p55 | . | . | | .... |
| Protein: | | p18  p24  p15 | p12  p66/51  p31 | p12 | p23 | .... |

The gag gene encodes for a precursor protein known as p55. This is subsequently cleaved during reproduction of the virus to yield three smaller proteins known as p18, p24, and p15.

The nucleotide sequences of the gag gene of HIV-1 strains, as well as that of HIV-2 and of SIV$_{MAC}$ have been published by Myers, G. et al (editors) "Human Retroviruses and AIDS. A Compilation and Analysis of Nucleic Acid and Amino Acid Sequences", Theoretical Biology and Biophysics Group T-10, New Mexico" (1987). These sequences, as well as the sequences of the corresponding proteins, have been published by Los Alamos National Database.

The gag proteins are located in the core of the virus. To date the main focus of research has concentrated on the development of an envelope vaccine because there aredata which indicate that certain retroviral envelope proteins confer antibody-mediated protection against viral challenge in animals. However, so far HIV-1 envelope vaccines have failed to protect chimpanzees against infection (see, for example, Hu S-L., et al "Nature", 328, pages 721 to 723).

In a paper entitled "Prediction of antigenic determinants and secondary structures of the major AIDS virus proteins", by Sternberg M.J.E., Barton G.J., Zvelebil M.J., Cookson J and Coates A.R.M., FEBS Letters, 218, No. 2, June 1987, pages 231 to 237, it is proposed that cytotoxic T-cells may play an important role in resistance to HIV-1 as this type of cell induces protection in chronic viral infection. It states:

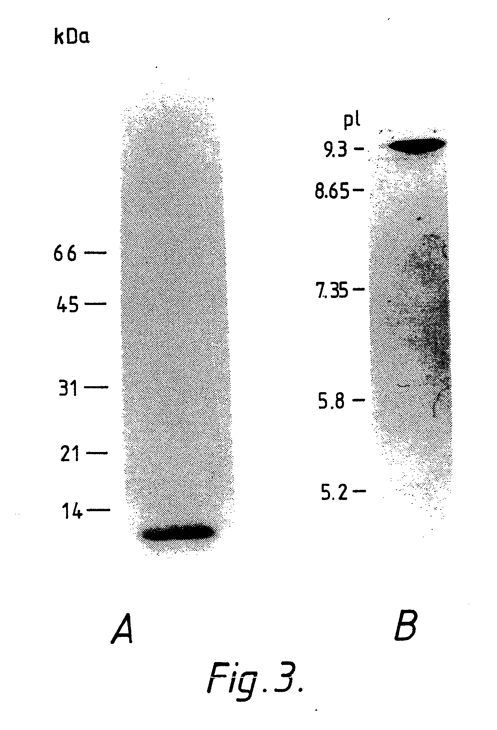

"The results of the gag polyproteins (Figure 3) have been presented elsewhere. In outline p17 (1-132) and p24 (133 to about 373) belong to the α/α class of proteins. The last 40 residues of p17 and residues 220-260 of p24 are probably exposed loops that would be ideal candidates for stimulating a B-cell antigenic response."

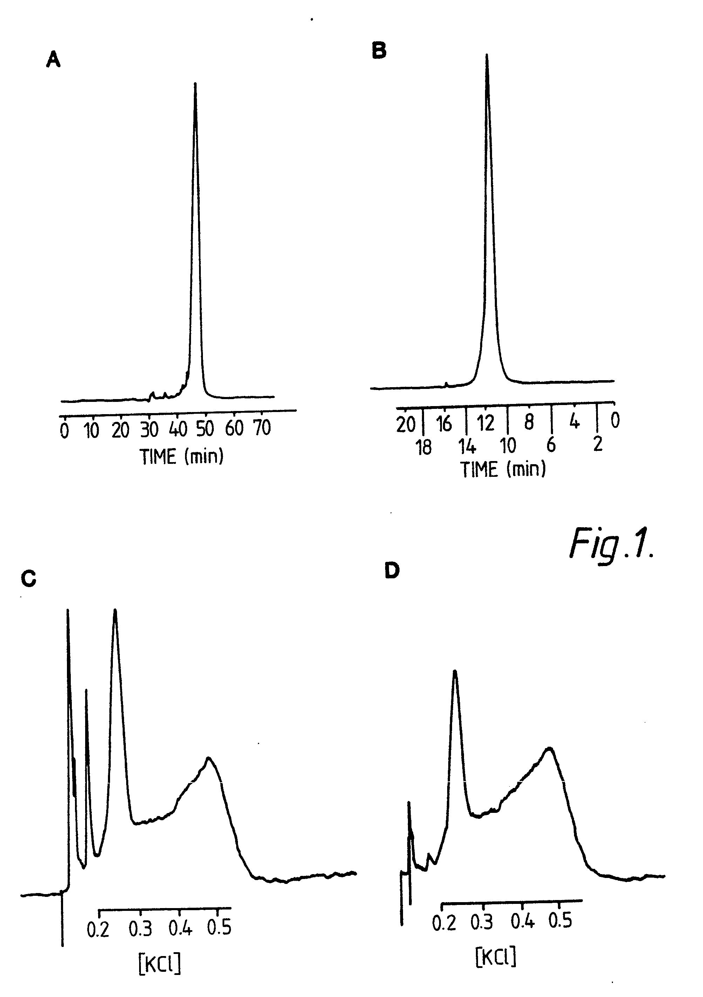

Figure 3 of the paper provides an analysis of the gag gene and indicates predicted positions of T-epitopes. (It should be noted that the numbering used to identify the amino acid residues in that paper differ from the numbering used in this specification, which corresponds to that given in Figure 1 of a later paper by Zvelebil M.J.J.M., Sternberg M.J.E., Cookson J., and Coates A.R.M., FEBS Letters, 242, No. 1, December 1988, pages 9 to 21). The paper in the June 1987 issue of FEBS Letters goes on to propose that:

"... in gag, which has a more conserved sequence than env, a short peptide containing residues 288-304 (GPKEPFRDYVDRFYKTL) meets this condition and may be effective as a synthetic vaccine."

The reference to the results of the gag polyproteins having been presented elsewhere is a reference to a paper by Coates A.R.M., Cookson J., Barton G.J., Zvelebil M.J., and Sternberg M.J.E., "Nature", 326, 9th April 1987, pages 549 to 550. This also mentions the short peptide containing residues 288-304 "which is the only 15-30 residue section containing both B and T epitopes in a sequence conserved region."

It would be extremely desirable to develop a vaccine against AIDS, particularly a vaccine that is effective in man. However, in spite of an increasing amount of research effort by numerous groups around the world over a number of years, the search for a vaccine has so far been unavailing.

The present invention accordingly seeks to provide novel synthetic polypeptides which can be used as a basis for a potential vaccine against AIDS and which can be manufactured in significant quantity and with sufficient purity for further study of the AIDS virus and potential methods of treatment. It also seeks to provide particularly T epitopes (but also B epitopes) of the p24 protein of the AIDS virus for use as potential components of an AIDS vaccine. It also seeks to provide a vaccine that is effective against all strains of HIV-1 and against HIV-2. It further seeks to provide a vaccine which is capable of being tested against $SIV_{MAC}$ in monkeys. It further seeks to provide synthetic polypeptides which can provide useful information on the immunogenicity and the specificity of the immune response to HIV proteins.

According to one aspect of the present invention we provide the synthetic polypeptides of the following formula:

$$\begin{array}{ccccc} 280 & 290 & 300 & 310 & 320 \end{array}$$

LNKIVRMYSPTSILDIRQGPKEPFRDYVDRFYKTLRAEQASQEVKNWMTETL

$$\begin{array}{cccc} 330 & 340 & 350 & 360 \end{array}$$

LVQNANPDAKYILKALGPAATLEEMMTAAQGVGGPGHKARVL-X     (I)

where X represents the carboxylic acid group of the L residue 367 or the polypeptide sequence:

$$370$$

AEAMSQVTNS

as well as polypeptides of the formula (I) wherein the residues 334 and 354 are changed from alanine to the residue of a neutral amino acid other than alanine or to a residue of cysteine.

Such synthetic polypeptides correspond to a homologous region of the p24 protein of a human immunodeficiency virus type 1 (HIV-1) at or adjacent its C-terminal end or are closely related thereto. The invention further relates to synthetic polypeptides which are analogously based on a similar homologous region of another strain of HIV-1 or of HIV-2 or of $SIV_{MAC}$. Also within the scope of the present invention are certain synthetic polypeptides of shorter chain length than those of the formula (I) but which correspond to a portion thereof or are related thereto. In addition the invention provides synthetic polypeptides which are derived from a polypeptide according to the invention, whether of the formula (I) or of shorter chain length, by substitution of one or more amino acid residues by residues of other amino acids, which do not affect its properties in vivo or in vitro, by addition of one or more amino acid residues at either end or at one or more points intermediate its ends, or by deletion of one or more amino acid residues therefrom, in each case without significantly altering its antigenic properties in vivo or in vitro or its properties as a diagnostic agent.

In another aspect of the invention there is provided synthetic polypeptides which consist of or which contain at least 5 consecutive residues found in the following polypeptide sequence:
LEEMMTAAQGVG     (II).

The invention further contemplates synthetic polypeptides which consist of or which contain the polypeptide sequence:
LEEMMTAAQGVG     (II).

Also provided in accordance with the invention are synthetic polypeptides which consist of at least 75 aminoacid residues and which contain the polypeptide sequence:

LEEMMTAAQGVG    (II).

Such polypeptides preferably contain no more than about 120 amino acid residues in total.

We also provide synthetic polypeptides which consist of or which contain at least 5 consecutive residues found in the polypeptide sequence:

LEEMMTAΦQGVG    (III),

where Φ represents an alanine residue or a residue of another neutral amino acid or a residue of cysteine.

The invention also provides polypeptides of the following formula:

θ-LEEMMTAΦQGVG-δ    (IV),

where θ represents a polypeptide sequence containing up to 80 amino acid residues, Φ represents an alanine residue, a residue of a neutral amino acid other than alanine, or a residue of cysteine, and δ represents a polypeptide sequence containing up to 25 amino acid residues. Preferably θ represents a polypeptide sequence containing up to 73 amino acid residues and δ represents an amino acid sequence containing up to 19 amino acid residues. In such polypeptides θ and δ preferably represent portions of the p24 peptide found in a human immunodeficiency virus of the type 1 (HIV-1) or type 2 (HIV-2) or in the virus SIV$_{MAC}$.

The invention also relates to T-cell epitopes being polypeptide fragments included within a polypeptide according to the invention.

Throughout this specification the following abbreviations are used:-

| Amino Acid | Three Letter Code | One Letter Code |
|---|---|---|
| Alanine | Ala | A |
| Arginine | Arg | R |
| Asparagine | Asn | N |
| Aspartic Acid | Asp | D |
| Asparagine or Aspartic Acid | Asx | B |
| Cysteine | Cys | C |
| Glutamine | Gln | Q |
| Glutamic Acid | Glu | E |
| Glutamine or Glutamic Acid | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The polypeptide of formula (I) wherein X represents the polypeptide sequence AEAMSQVTNS, contains 104 amino acid residues and so is sometimes hereinafter called "the 104-mer". The 104-mer corresponds to the sequence of the C-terminal half of the core protein, p24 of the human immunodeficiency virus type 1$_{LAV}$ (HIV-1$_{LAV}$), except that the cysteine residues 334 and 354 of natural p24 are replaced by alanine. This change obviates potential complications caused by the formation of disulphide links between polypeptide chains.

In formula (I) the numerals (i.e. 280 etc) appearing above the polypeptide sequence indicate the relevant equivalent position in the polypeptide sequence of the p24 protein of the HIV-1 virus. However in formula (I) the residue 334 is cysteine in the HIV-1 virus, and not alanine, as also is the residue at position 354. The sequence for the p24 protein of the gag gene is available from the Protein Information Resource Databank, code FOVWLV.

4

The invention further relates to the following polypeptides which are present as epitopes in the polypeptide of the formula (I):

(i)

$$360 \quad\quad 370$$

$$\text{QGVGGPGHKARVLAEAMSQVTNS} \quad\quad\quad (V)$$

(hereinafter called "epitope PG1");

(ii)

$$280 \quad\quad 290$$

$$\text{LNKIVRMYSPTSILDIR} \quad\quad\quad (VI)$$

hereinafter called "epitope PG2");

(iii)

$$320$$

$$\text{QASQEVKNWMTETLL} \quad\quad\quad (VII)$$

(hereinafter called "epitope PG3");

(iv)

$$330 \quad\quad 340$$

$$\text{ANPDAKYILKALGPAAT} \quad\quad\quad (VIII)$$

(hereinafter called "epitope PG4");

(v)

$$350 \quad\quad 360$$

$$\text{GPAATLEEMMTAAQGVGGP} \quad\quad\quad (IX)$$

(hereinafter called "epitope PG5"); and

(vi)

$$300$$

$$\text{QGPKEPFRDYVDRFY} \quad\quad\quad (X).$$

(hereinafter called "epitope P-II").

The invention further provides the polypeptide of the formula:

PGGVGQAATMMEELTAAPG (XI).

Using the method of Barton, G.J. & Sternberg, M. J. E., J. Mol. Biol, (1987), 198, pages 327 to 337, it is possible to investigate the extent of inter-strain variation between various HIV-1 isolates. Predictions can then be made of T-cell epitopes based on various criteria. In particular it is possible to analyse conservation of sequences within the polypeptides from the HIV-1 strains, from HIV-2 and from SIV$_{MAC}$. It is also possible to analyse predicted antigenic epitopes by introducing appropriate quantitative measures. This enables epitopes to be predicted which are at least twelve residues long as peptides of this length have been shown frequently to contain an epitope.

Two algorithms can be used to predict T-cell epitopes. The first algorithm, which has been described by DeLisi, C. & Berzofsky, J. A. (1985), Proc. Natl. Acad. Sci. USA. 82, Pages 7048 to 7052, calculates the amphipathicity of a section eleven residues long and regions with a periodicity that corresponds to α-helices and identifies such sections as possible T-cell epitopes. The second procedure is that of Rothbard, J. B. and Taylor, W. R. (1988), The EMBO Journal, 7, pages 93 to 100. This second algorithm uses a general motif found from an analysis of T-cell epitopes. The protein sequences is scanned for a linear pattern composed of a charged residue or a glycine in the first position, followed by 2 or 3 hydrophobic amino acids and terminated by another charged or polar residue.

Upon ordering the T-epitopes in rank order, according to their inter-strain percentage identity, the results set out in Table 2 below are obtained.

TABLE 2

| HIV-1 | | | | | HIV-2 | | | | | | SIV-MAC | | | | | |
|---|---|---|---|---|---|---|---|---|---|---|---|---|---|---|---|---|
| Epitope Numbers | Epitope Sequence | DB | RT | %SI | Epitope Numbers | DB | RT | Ov %1-2 | Ext %1-2 | HIV-1 %1-2 | Epitope Numbers | DB | RT | Ov %1-S | Ext %1-S | HIV-1 %1-S |
| 347-359 | LEEMMTACQGVG | Y | Y | 100 | 346-357 | Y | Y | 91 | 92 | 92 | 346-357 | Y | Y | 91 | 92 | 92 |
| 73-84 | EELRSLYNTVAT | Y | | 92 | 57-80 | Y | Y | 62 | 53 | 58 | 70-82 | Y | | 75 | 73 | 67 |
| 156-167 | NAWVKVVEEKAF | | Y | 92 | 156-167 | | Y | 83 | 83 | 83 | 156-167 | | Y | 75 | 75 | 75 |
| 170-181 | EVIPMFSALSEG | Y | | 92 | 170-181 | Y | | 75 | 75 | 75 | 170-181 | Y | Y | 67 | 67 | 67 |
| | | | | | | | | | | | 178-189 | | Y | 100 | 58 | 67 |
| 198-209 | QAAMQMLKETIN | Y | | 92 | 198-209 | Y | Y | 67 | 67 | 67 | 198-209 | Y | Y | 58 | 58 | 58 |
| 215-226 | WDRVHPVHAGPI | | Y | 92 | gap | | | | | | gap | | | | | |
| 273-284 | GLNKIVRMYSPT | | Y | 92 | 273-284 | | Y | 75 | 75 | 75 | 273-284 | | Y | 75 | 75 | 75 |
| 335-346 | KTILKALGPAAT | Y | Y | 92 | 331-342 | | Y | 62 | 62 | 50 | 331-342 | | Y | 62 | 62 | 50 |
| 365-376 | RVLAEAMSQVTN | Y | Y | 92 | 365-376 | Y | Y | 42 | 42 | 42 | 365-376 | Y | Y | 33 | 33 | 33 |
| 291-307 | QGPKEPFRDYVDRFYKT | Y | Y | 88 | 295-307 | Y | Y | 77 | 82 | 82 | 295-307 | Y | Y | 77 | 82 | 82 |
| 96-107 | DTKEALDKIEEE | Y | Y | 83 | 96-107 | Y | | 25 | 25 | 25 | 96-107 | Y | | 25 | 25 | 25 |
| 187-198 | LNTMLNTVGGHQ | Y | | 83 | 184-195 | Y | | 67 | 71 | 67 | | | | | | 67 |
| 207-218 | TINEEAAFWDRV | | Y | 83 | 207-218 | | Y | 75 | 75 | 75 | 207-218 | | Y | 67 | 67 | 67 |
| 260-271 | IPVGEIYKRWII | | Y | 83 | | | | | | 67 | | | | | | 75 |
| 311-322 | FQASQEVKNWMT | Y | | 83 | 311-322 | | Y | 67 | 67 | 67 | 311-322 | | Y | 67 | 67 | 67 |
| 415-426 | KGCWKCGKEGHQ | Y | | 83 | 417-428 | Y | Y | 80 | 71 | 75 | 423-434 | | Y | 25 | 60 | 67 |
| 479-491 | SQKQEPIDKELY | | Y | 83 | gap | | | | | | gap | | | | | |

EP 0 356 007 A2

In Table 2 the abbreviation "DB" indicates that the algorithm of DeLisi and Berzofsky was used, whereas the abbreviation "RT" indicates that the algorithm of the procedure of Rothbard and Taylor was used. The abbreviation "%-SI" indicates the inter-strain percentage identity. If an epitope is found in corresponding region in HIV-2 or $SIV_{MAC}$, then their position numbers are given (Epitope Numbers). The abbreviation Ov %1-2 denotes the percentage identity between the overlapping epitope predictions between HIV-1 and HIV-2, and Ov %1-S the corresponding percentage identity between HIV-1 and $SIV_{MAC}$. Ext %1-x gives the percentage identity for all predicated epitope positions of that region, and HIV-1 %1-x denotes the percentage identity between the HIV-1 epitopes and the corresponding sequence in either HIV-2 (i.e. x = 2) or $SIV_{MAC}$ (i.e. x = S).

Epitope PG1 includes epitope number 335-346. The epitope PG2 of formula (III) includes most of the epitope number 273-284. The epitope PG3 of formula (IV) includes most of the epitope number 331-322. The epitope PG4 of formula (V) includes epitope number 335-346. The epitope PG5 of formula (VI) includes the epitope number 347-359, except that A replaces C at position 354. The epitope P-II of formula (VII) includes the epitope number 291-307.

The results plotted in Table 2 show that the gag proteins, p17, p24 and p15 contain seventeen epitopes which are at least 83% conserved between 10 different HIV-1 strains studied. Most, i.e. 13 out of 17 of these epitopes, are concentrated in p24, whilst only two are found in each of p15 and p17.

The epitopes of formulae (V) to (X) appear in the C-terminal half of p24. This C-terminal half of p24 is particularly interesting since it contains the only predicted T-epitope in gag which is 100% conserved between the ten HIV-1 strains examined and is also 92% conserved between HIV-1 and HIV-2, and also between HIV-1 and $SIV_{MAC}$.

The synthetic polypeptides of the invention, such as those of the formulae (I) to (XI) can be produced by chemical synthesis using preferably t-Boc chemistry. Alternatively they can be produced using recombinant DNA technology.

The synthetic polypeptides of the invention, such as those of one of the formulae (I) to (XI), can be used singly or as a mixture of two or more thereof as a component of a vaccine intended to provide protection against the human immunodeficiency virus type 1 (HIV-2) and/or type 2 (HIV-2) and/or against the $SIV_{MAC}$ virus. Such a vaccine may further include one or more natural or synthetic polypeptides which have a beneficial effect in protecting a human being or other organism, such as a monkey, against a virus of the type HIV-1, HIV-2 or $SIV_{MAC}$. Such a vaccine may contain any of the usual adjuvants. The vaccine may be stored preferably at low temperature, in ampoules containing one or more unit dosages. It may be subjected to freeze-drying prior to storage. A typical unit dosage of vaccine contains from about 5µg to about 1 mg of a synthetic polypeptide or polypeptides according to the invention. Normally it will be expedient to administer a dose of the vaccine by injection. It is envisaged that it will typically suffice to inject the subject up to 3 times to provide protection against infection by the relevant virus, i.e. HIV-1, HIV-2 or $SIV_{MAC}$. Booster injections may be required at intervals in order to maintain protection.

The synthetic polypeptides of the invention such as those of formula (I) can be used to raise antibodies which recognise epitopes on viral proteins of the HIV-1 virus, such as the proteins p24 and p55. They can further be used to produce monoclonal antibodies. Such antibodies can be used for diagnostic purposes for detecting the presence of viral antigen in a serum or in a tissue culture using conventional techniques such as immunofluorescence. They can further be used for the production of anti-idiotypes.

The synthetic polypeptides of the present invention can also be used as diagnostic agents. For example they can be used in ELISA tests or in the liquid phase.

The invention is further illustrated in the following Examples. In the Examples the following abbreviations are used:

| | |
|---|---|
| HIV-1$_{LAV}$ = | Human immunodeficiency virus type 1$_{LAV}$ |
| t-Boc = | t-butoxycarbonyl |
| PAGE = | Polyacrylamide gel electrophoresis |
| SDS = | Sodium dodecyl sulphate |
| ELISA = | Enzyme-linked immunosorbent assay |
| B and T = | B and T lymphocyte determined |
| DMF = | Dimethyl formamide |
| DCM = | Dichloromethane |
| HF = | Hydrogen fluoride |
| F.a.b. = | Fast atom bombardment |
| PBS = | Phosphate buffered saline |
| PBS/T = | Phosphate buffered saline containing Tween 20 |
| FCS = | Foetal calf serum |
| BSA = | Bovine serum albumin |
| IFA = | Freund's incomplete adjuvant |

Example 1

Chemical synthesis of 104-mer p24 core protein.

Data on the amino acid sequence of the provirus, HIV-1$_{LAV}$ published by Wain-Hobson, S., Sanigo, P., Danos, O., Cole, S. and Alizon, A. (1985) Cell 40, 9-17 was utilised in the synthesis of the 104-mer peptide. The peptide of formula (I) in which X represents:

370

**AEAMSQVTNS**

(hereinafter called !the 104-mer" or "the 104-mer gag p24 polypeptide") was grown on acid stable phenylacetamidomethyl resin using an ABI 430-A synthesiser (Applied Biosystems) and a chemical protocol based on the Boc-amino acid chemistry. The resin contained the first protected amino acid of the sequence (Ser) with a substitution factor of 0.76 meq/g; 0.66 g of amino acid resin was used which corresponded to 0.5 meq Boc-Ser (O-benzyl). Some improvements over the conventional approach to solid-phase protein synthesis, which aimed at increasing the average coupling efficiency to at least 99.5%, were as follows:

The N-protected amino acids were pre-activated in dichloromethane (DCM) to the corresponding anhydride. This was followed by the exchange of DCM for dimethyl formamide (DMF), which is a solvent with better resin swelling properties than DCM. Double coupling procedures were applied throughout the synthesis and flow washes replaced the conventional "washing steps" between two successive chemical reactions. This reduced the contact time between the growing peptide and the potentially destructive solvents and reagents. The time of the double coupling cycle was thus reduced to an average 105 min. The amino acid incorporation performances were monitored by the quantitative ninhydrin test using the procedure described by Sarin, V.K., Kent S.B.H., Tam J.P., and Merrifield R.B., Anal. Biochem., (1981), 117, 147-157.

For amino acid side chain protection the most acid stable groups (known at the time of the synthesis) were used; these were as follows:

1) benzyl ester and ether for Asp/Glu and Ser/Thr respectively;

2) formyl group and dinitrophenyl for Trp and the imidazole ring of His;

3) 2-CL-carbobenzoxy and o-2-Br-carbobenzoxy for Lys and Tyr respectively;

4) tosylate group for the Arg side chain.

The two Lys residues at 334 and 354 were replaced by the amino acid alanine. Protected amino acids, solvents and reagents were of the highest possible grade (A.B.I., Warrington, U.K.) to reduce to a minimum the adverse effect of impurities.

The coupling reactions were carried out using a fourfold excess of Boc-amino acid (twofold excess in the anhydride). In the case of Gln, Asn and Arg fourfold excess of the corresponding protected amino acid was derivatised to the hydroxy-benzotriazole active ester prior to coupling, in a DMF/DCM mixture (9:1, v/v), and the resulting solution transferred to the reaction vessel without further solvent exchange.

The synthesis was carried out with no interruption except when changing of the solvents and reagents was needed (usually every 14 residues) and for the taking of resin-peptide samples. The total synthesis time was thus just over seven days, and approximately 3 g of peptide-resin was obtained.

Prior to cleavage from the resin support the dinitrophenyl group was removed from the His side-chain by two successive thiolic treatments of half hour each using 20% 2-mercaptoethanol, and 10% diisopropylethylamine in DMF. This was followed by extensive washings with DMF and DCM and treatment with 60% trifluoroacetic acid in DCM for the deprotection of the last t-Boc amino acid of the sequence (half hour).

Finally, a low-high hydrogen fluoride (HF) treatment in the presence of scavengers (see the paper by Tam, J.P., Heath, W.F. and Merrifield, R.B. (1983). J. Am. Chem. Soc. 105, 6442-6455) was used to remove the remaining side chain protecting groups and for the cleavage of the peptide-resin anchoring bond. In the low part of the HF treatment typically 500 mg of peptide resin was suspended in 6 ml of dimethylsulphide containing 0.8 ml of p-cresol and 0.2 ml of p-thiocresol. After distillation of 3 ml of HF, the reaction was allowed to proceed for one hour at 0°C. The HF was then removed in vacuo, the dimethylsulphide by filtration and the peptide resin dried. The latter was then subjected to the high HF (10 ml) treatment for one hour at 0°C using 0.8 ml of p-cresol and 0.2 ml of p-thiocresol as the scavengers. After removal of HF the peptide was precipitated in diethylether, dissolved in 5% acetic acid and lyophilised. Six short p24 peptides which corresponded to parts of the sequence of the 104-mer (274-290, 291-305, 312-326, 330-346, 342-358, 355-377) were similarly synthesised by conventional solid-phase synthesis but without the improvements described above. After reverse phase high performance chromatography purification, the molecular mass and sequence of these peptides (epitopes PG1, PG2, PG3, PG4, PG5 and P-II) were obtained by F.a.b. mass spectrometry. Amino acid analysis was also performed.

Purification of the synthetic protein

Crude 104-mer peptide material (100 mg) was dissolved in about 10 ml of a solution of 6M guanidine HCl, 50 mM Tris acetate, 20% v/v 2-mercaptoethanol, pH 8.5, and stirred at room temperature for two hours to remove residual DNP groups. The solution was then transferred to dialysis tubing (Spectra/Por 6, $M_r$ cut-off 6000, Spectrum Medical Industries Inc., Los Angeles), and dialysed against 150 mM ammonium acetate buffer, pH 8.3, for 2 days at 4°C. The dialysed solution was then lyophilised and the powder material thus obtained further purified by semi-preparative reverse phase HPLC (C4, Vydac). The broad peak eluting between 70 and 80% acetonitrile (42-50 mins) was collected and subjected to lyophilisation followed by size exclusion high performance chromatography (TSK GW2000) in 20 mM Tris-HCl pH 5.2, 2 mM EDTA and 0.1 M KCl. During the latter chromatography step material eluting between 10.5 and 12 minutes was collected. The last purification step involved the use of strong cationic exchange gradient FPLC (Mono-S, pharmacia), in solvent A as for the size exclusion chromatography and in solvent B (0%-100% in 1½ hours) which was the same except the concentration of KCl was increased to 1 M.

Chemical characterisation of the purified 104-mer

Purified polypeptide (1 mg) was subjected to acid hydrolysis (constant boiling HCl in the presence of phenol and 2-mercaptoethanol) at 110°C for 24 hours. Amino acid analysis was performed with a pharmacia LKB Alpha Plus analyser. The results are listed in Table 3.

TABLE 3

| Amino acid analysis of 104-mer | | |
|---|---|---|
| | Expected | Found |
| Asp + Asn | 9 | 9.5 |
| Thr | 8 | 8.6 |
| Ser | 5 | 4.9 |
| Glu + Gln | 13 | 13.5 |
| Pro | 6 | 6.5 |
| Gly | 6 | 6.2 |
| Ala | 12 | 12.7 |
| Val | 7 | 6.9 |
| Met | 5 | 5.6 |
| Ile | 4 | 2.8* |
| Leu | 9 | 8.9 |
| Tyr | 3 | 2.5 |
| Phe | 2 | 1.8 |
| Lys | 7 | 7.1 |
| His | 1 | 0.9 |
| Arg | 6 | 6.1 |

The amino acid analysis is consistent with the expected amino acid composition with the sole exception of Ile (2.8 residues found, expected 4). Discrepancies in the case of the bulky Ile are often experienced.

The single tryptophan residue does not appear in Table 3 because it is destroyed during the HCl hydrolysis step. The whole purified peptide preparation was then tested for homogeneity in SDS-PAGE (20% homogeneous gel, pharmacia) and in gel isoelectrofocusing (Ampholine Pagplate, pH 3.5-9.5) using the Pharmacia Phast System. The sequence of the N-terminal 40 amino acids and the C terminal 22 amino acids of the purified synthetic peptide was determined by using an Applied Biosystems amino acid sequencer (477A) and with F.a.b., mass spectrometry (VGZABSE), respectively. Tryptic digestion and F.a.b. mass spectrometry peptide mapping were then used to identify the remainder of the sequence. 1 mg of 104-mer was incubated at $37°$ C in 1 ml of NaHCO$_3$ buffer (pH = 8.5) containing 20μg of trypsin. After 2 hours the reaction was quenched by the addition of aqueous HCl (pH 3) and the peptide fragments separated by C-18 reverse phase high performance chromatography (gradient: from 20% to 100% B in 60 mins; A = 0.1% trifluoracetic acid, B = 80% acetonitrile, 0.1% trifluoracetic acid, B = 80% acetonitrile, 0.1% trifluoracetic acid). Purified fragments were then subjected to F.a.b. mass spectrometric analysis and the sequence identified by a computer search programme designed to screen the parent peptide sequence for a molecular mass corresponding to the found mass spectrometry molecular ion.

In the above described purification procedure the dialysed protein yielded one major peak on C4 reverse phase chromatography (Figure 1A). After size exclusion chromatography, again one peak was observed (Figure 1B) which eluted after 11 minutes. In a calibration, run cytochrome C (Molecular mass about 13,000 daltons) also eluted after 11 minutes and so the synthetic material eluted at a time which was consistent with the expected molecular mass of 11,507 daltons.

The cationic exchange HPLC. profile in Figure 1C shows synthetic p24 eluting between a KCl concentration of 0.2M and 0.3M. After the cationic exchange (Figure 1D) chromatography, one major peak was observed which eluted at this concentration and this peak contained the 104-mer peptide. The broad peak between 0.4-0.5 M KCl is due to base-line drift. The four step purification procedure typically yielded 20 mg of homogeneous synthetic polypeptide. This corresponded to 20% yield w/w of the crude material.

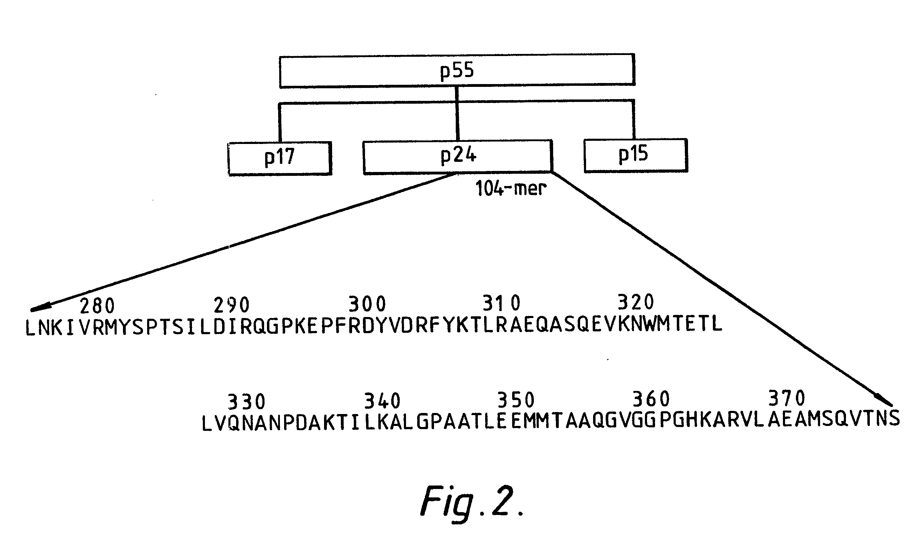

Figure 2 illustrates the relationahip between the HIV-1 precursor p55 which is cleaved by viral protease to yield p17, p24 and p15 (p9/6). The sequence of the 104-mer is the same as that of the corresponding C terminal half of p24 except that alanine was substituted for cysteine at positions 334 and 354.

The purified synthetic 104-mer had a molecular mass of about 11,000 daltons by sodium dodecyl sulphate gradient-polyacrylamide gel electrophoresis (SDS-PAGE) (Figure 3A) which was consistent with the M$_r$ of 11,507 calculated from the 104-mer sequence. The protein migrated as a single homogeneous band.

The homogeneity of the synthetic material was further tested by iso-electrofocusing gel chromatography. In Figure 3B, synthetic 104-mer p24 focussed as a single band with a pI of about 9.4. This was in agreement with the pI expected from the basic and acid residue composition of the polypeptide.

Gas-phase peptide sequencing of the purified peptide was carried out and thirty-nine successive cycles of the Edman degradation were performed before the signal became too small for unequivocal determination; the results of the microsequencing which are set out in Table 4 were consistent with the sequence expected between residues Leu-1 and Ala-40.

Table 4

| Gas-phase sequencing of synthetic 104-mer | | | | | |
|---|---|---|---|---|---|
| Cycle No. | Found | Expected | Cycle No. | Found | Expected |
| 1 | Leu (649) | Leu | 21 | Lys (96) | Lys |
| 2 | Asn (457) | Asn | 22 | Glu (59) | Glu |
| 3 | Lys (472) | Lys | 23 | Pro (67) | Pro |
| 4 | Ile (494) | Ile | 24 | Phe (62) | Phe |
| 5 | Val (433) | Val | 25 | Arg (5) | Arg |
| 6 | Arg (139) | Arg | 26 | Asp (41) | Asp |
| 7 | Met (546) | Met | 27 | Tyr (45) | Tyr |
| 8 | Tyr (325) | Tyr | 28 | Val (46) | Val |
| 9 | Ser (174) | Ser | 29 | Asp (57) | Asp |
| 10 | Pro (250) | Pro | 30 | Arg (3) | Arg |
| 11 | Thr (47) | Thr | 31 | Phe (33) | Phe |
| 12 | Ser (53) | Ser | 32 | Tyr (26) | Tyr |
| 13 | Ile (163) | Ile | 33 | Lys (59) | Lys |
| 14 | Leu (187) | Leu | 34 | Thr (8) | Thr |
| 15 | Asp (62.5) | Asp | 35 | Leu (39) | Leu |
| 16 | Ile (148) | Ile | 36 | Arg (4) | Arg |
| 17 | Arg (53) | Arg | 37 | Ala (9) | Ala |
| 18 | Gln (149) | Gln | 38 | Glu (7) | Glu |
| 19 | Gly (110) | Gly | 39 | Gln (11) | Gln |
| 20 | Pro (63) | Pro | | | |

In Table 4 the numbers in parenthesis correspond to yields of phenylthiohydantoin derivatives in p.mole. The sample used for the sequencing contained 75 p.mole of synthetic polypeptide.

F.a.b. mass-spectrometry was used to identify both the molecular mass and the sequence of the 83-104 fragment. This was obtained from a sample of peptide-resin taken after the incorporation of the 23rd reside. The molecular mass and the sequence were consistent with that expected for the peptide. Tryptic digestion and F.a.b. mass spectrometry peptide mapping of purified fragments showed that the remainder of the molecule contained the expected sequence. Fragments 279-290, 277-298, 299-306, 319-335 and 340-363 had a molecular mass which corresponded to the expected sequence. The amino acid sequences 314-318 and 336-339 were not identified during the peptide mapping or sequencing procedures.

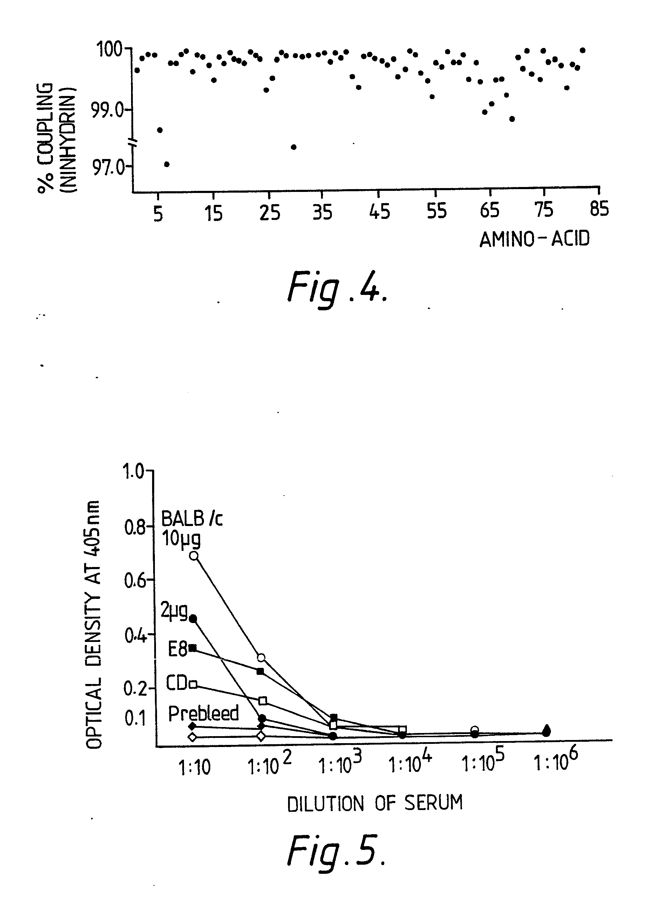

During synthesis of the 104-mer the average incorporation measured for the first 86 residues of the 104-mer was 99.5%, as shown in Figure 4, which corresponded to a predicted yield, before the final deprotection step, of 60% of molecules with the correct sequence. Using the procedures described above 3 g of resin-peptide material were obtained just over seven days after initiation of the chain assembly.

Example 2:

Antibodies

Animal sera

Rabbits (New Zealand white, Ranch Rabbits Farm, England) and inbred (BALB/c) mice (Olac Farm, Oxon, England) were injected with the purified 104-mer emulsified in Freund's incomplete adjuvant (IFA), (Sigma Chemical Co., Poole, England) and each animal was inoculated once subcutaneously with either 50, 10 or 2μg of the immunogen, followed by a similar boosting dose after 1 month with the peptide in IFA corresponding to the priming dose they had been given. A similar schedule was followed for mice immunised with the short peptides except that a dose of 50 μg per injection was used. Blood of the animals was collected 10 days post challenge, and the sera were heat inactivated at 56°C for 30 minutes before being stored at -70°C until testing.

## Human sera

Human sera were collected from HIV antibody negative healthy laboratory workers (controls) and from HIV antibody positive haemophiliacs by venous puncture after obtaining consent from the patients. Sera were treated and stored as described above.

## Monoclonal antibody generation.

This was essentially carried out as previously described by Haaheim, L.R. and Lund, H. (1984) Acta. Path. Microbiol. Scand. 92, 325-330, using lymphocytes from BALB/c mice which were immunised with 10μg of 104-mer in IFA as described above. Four weeks after the second injection animals were given a similar dose intravenously and spleen cells were harvested after 3 days. NSO myeloma cells were used as fusion partner. The hybridoma supernatants were screened 14 days post-fusion against the 104-mer by ELISA, and a representative panel of hybridomas were cloned at limiting dilution.

## Antibody testing

## Enzyme-linked immunosorbent assay (ELISA).

Lyophilised 104-mer peptide was dissolved in distilled water at 1 mg/ml and diluted in 0.01 M phosphate buffered saline (PBS), pH 7.2, to 10μg/ml. One hundred μl of the peptide solution was then aliquoted into each well of the 96 well ELISA plates (Costar type 3590, M.A., U.S.A.). The plates were sealed and kept at 4°C overnight.

After aspiration of the antigen solution from the wells the plates were blocked with 300 μl PBS containing 2% w/v bovine serum albumin (Sigma) by leaving the plates for 1-2 hours at room temperature. The plates were washed three times to remove free peptide with wash buffer (PBS) containing 0.05% Tween 20 (Sigma) (PBS/T)) and blotted with paper towels. Animal and human sera were diluted in PBS containing 1% bovine serum albumin, and 100μl of the sample was added to the peptide-coated wells. Binding of antibodies to the peptide was allowed to take place at room temperature for 1 hour. Unbound antibodies were removed by washing the plates four times with PBS/T. Fifty μl/well of anti-mouse (Daka, Denmark) or anti-rabbit (Sigma) immunoglobulin (in certain cases specific for IgG) horseradish peroxidase conjugates, appropriately diluted in PBS/T, was then added to detect the binding of the mouse or rabbit immunoglobulins to the peptide. Binding of human IgG was detected by adding 50μl of anti-human IgG alkaline phosphatase conjugate (Sigma) to the appropriate wells. After incubation at room temperature for 1 hour, free conjugate was removed by washing the plates five times in PBS/T and 100μl/well of appropriate enzyme substrate solution was added. After colour development was arrested, optical densities were read on a Titertek MultisKan spectrophotometer at 492 nm for peroxidase or 405 nm for alkaline phosphatase reaction.

## Immunoblot for the detection of serum antibody to HIV proteins.

Nitrocellulose strips containing Western blotted HIV-1 proteins after SDS-PAGE separation were obtained from Bio-Rad (Cat. No. 1971002). Immunoblotting was carried out essentially according to the manufacturer's instructions except that the peroxidase-labelled conjugates were from Dako (rabbit anti-mouse Ig; and swine anti-rabbit Ig). The substrate used was horse-radish peroxidase colour development reagent containing 4-chloro-1-naphthol (Bio-Rad).

Immunisation of BALB/c mice with the 104-mer gag polypeptide according to Example 1 led to the production of specific IgG antibodies.

The 104-mer polypeptide prepared according to Example 1 is immunogenic in mice and rabbits. Sera from mice bind to both synthetic 104-mer and to native p24. Human sera from HIV-1 infected individuals, as well as murine monoclonal antibodies which bind to natural p24, also react with the synthetic 104-mer in ELISA and in immunoblots. The 104-mer contains at least four distinct B epitopes as defined by monoclonal antibodies, polyclonal antibodies and short synthetic peptides. The synthetic polypeptide also contains T epitopes which stimulate the proliferation of murine helper T-cell lines and T-cell clones. Five distinct T epitope-containing regions have been located in the 104-mer. The T cell clones and lines proliferate in the presence of two strains of natural HIV-1.

It has also been shown that the synthetic 104-mer contains B-epitopes. Thus sera from mice immunised with synthetic 104-mer bind both to the synthetic 104-mer and also to native p24.

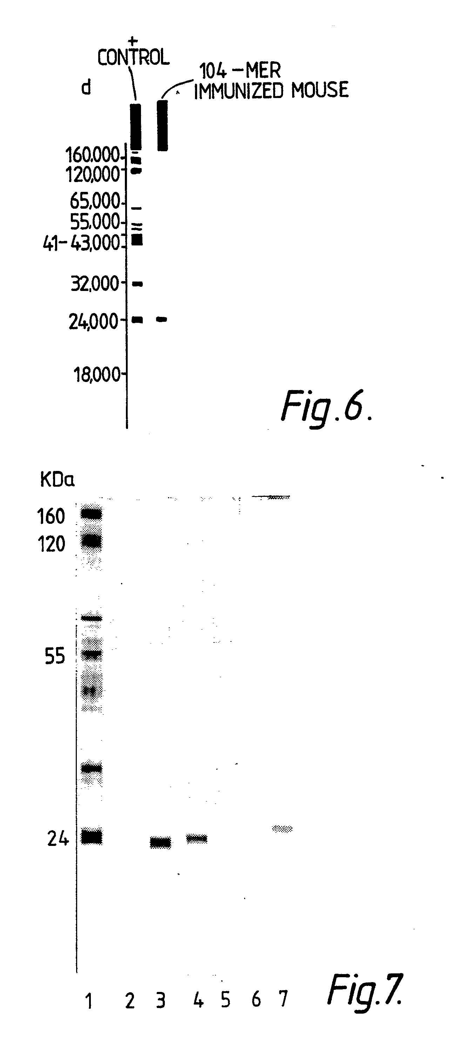

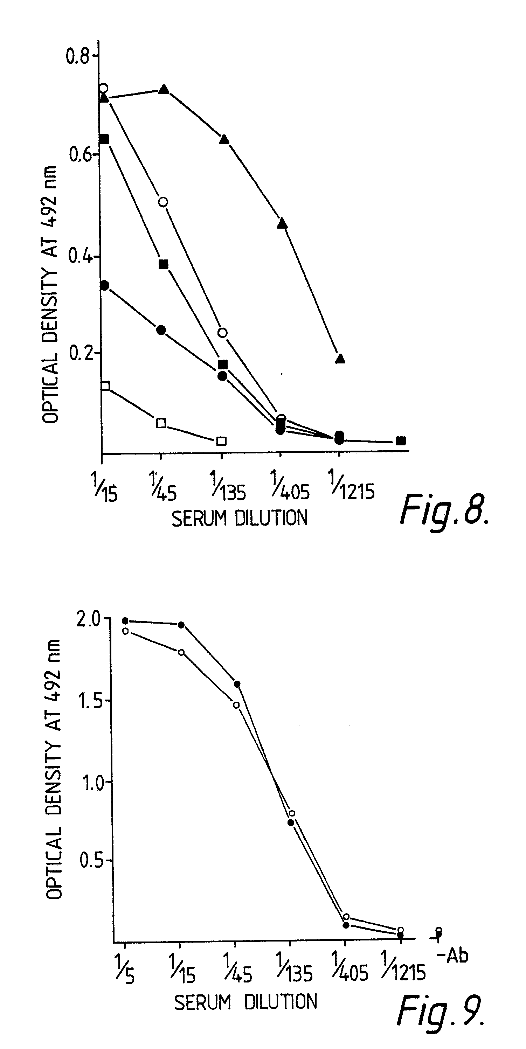

The 104-mer was immunogenic when injected into BALB/c mice. The polypeptide was administered in Freund's incomplete adjuvant at a dose of 2 μg or 10 μg. After two doses of antigen, the mice were bled and the sera were assayed for anti-p24 antibody in a solid-phase ELISA with the synthetic 104-mer adsorbed onto the bottom of plastic wells. The results of the ELISA are shown in Figure 1. Prebleeds gave an optical density reading of less than 0.05. The highest O.D. response of 0.7 was obtained with sera from mice which had received 10 μg of 104-mer. The sera from the synthetic 104-mer p24-immunised mice also bound to natural gag gene products. Immunoblots with native virus showed that the murine sera bound to both p24 and to the precursor p55 (see Figure 6).

Monoclonal antibodies were generated against the synthetic 104-mer. It was found that murine monoclonal antibodies which bind to natural p24 also react with the synthetic 104-mer.

The 104-mer specific antisera also reacted to viral p24 and to a lesser extent with p55 in Western blots, the results of which are shown in Figure 7. In Figure 7 lane 1 shows the reactivity of human serum from HIV-infected patient to the viral proteins. Reactivities of sera from mice which were unimmunised (lane 2) and immunised with the 104-mer (lane 3) are shown. Lane 4 shows the specificity of monoclonal antibody LH 104-A and lane 5 that of monoclonal antibody LH 104-B. Lanes 6 and 7 show the binding of serum from rabbits before and after immunisation with the 104-mer peptide respectively. Rabbit polyclonal antisera raised against the 104-mer perptide similarly recognised core proteins of HIV-1 in a Western blot (see Figure 7) and also bound to 104-mer in ELISA.

The murine monoclonal antibodies IE8G2 (E8) and CD12B4 (CD) which bind to natural p24 also bound to the 104-mer in the ELISA (Figure 5) and in immunoblots. The B epitope which is recognised by the monoclonal antibody IE8G2 is situated in the 269-311 region and that seen by CD12B4 is 312-346. It was found that the binding of IE8G2 is inhibited by a 15-mer peptide p18, from the MRC p24 series but CD12B4 was not inhibited by this peptide. These findings indicate that the E8 and CD monoclonal antibodies bind to different epitopes in the C-terminal half of p24.

Mice which were immunised with shorter p24 peptides (15-23 mers) produced antibodies which did not bind to p24 but reacted exclusively to the gag precursor p55. The strongest p55 reaction was observed with serum from peptide 274-290 (epitope PG-2). The weakest anti-p55 response was elicited by peptide 291-308 (epitope P-II).

It has further been found that sera from HIV-1 infected carriers which bind to natural p24 also bind to synthetic 104-mer.

These results suggest that the 104-mer is immunogenic and shares antigenic determinants with the viral core proteins p24 and p55.

Sera from HIV-1 infected haemophiliacs also bound to the 104-mer p24 polypeptide in ELISA and in immunoblots. Table 5 shows the comparison of results between ELISA with the synthetic 104-mer p24 and immunoblots (Biorad) with whole gag gene products for 4 sera from HIV infected haemophiliacs and 2 uninfected controls. The 4 sera from the infected patients bound to both synthetic 104-mer and to native p24. The reactivities of HIV-1 infected haemophilia sera against 104-mer in the solid phase ELISA are shown in Figure 8, in which the line ▲ - ▲ shows the results of patient 1, the line O-O shows those of patient 2, the line ● - ● those of patient 3, the line ■ - ■ those of patient 4, and the line □ - □ those of the sero-negative control. These results were expressed as mean O.D. 492 nm of duplicate assays performed at each serum dilution, standard error of mean SEM < 10%.

TABLE 5

| Binding of synthetic 104-mer p24 gag to human sera: comparison with natural p24 gag gene product. | | | |
|---|---|---|---|
| Synthetic 104-mer p24 | | | |
| Patient Laboratory Number | ELISA | Immunoblots | Immunoblots with whole p24 gag gene product: |
| | Mean Optical Density | | |
| | (1:45 dilution of serum) | (1:50 dilution of serum) | |
| 1 | 0.720 | + | + |
| 2 | 0.502 | + | + |
| 3 | 0.363 | + | + |
| 6 | 0.176 | + | + |
| Control (uninfected) | 0.040 | - | - |
| Control (uninfected) | 0.003 | - | - |

Inhibition of binding to p24 by 104-mer was observed with sera from patients number 1 and 6 (see Table 6). In the ELISA the serum binding of patient number 1 was reduced from an optical density reading of 1.94 (no soluble 104-mer) to 0.37 in the presence of 50 $\mu$g/ml of synthetic p24. Similarly the binding of serum from patient number 6 was reduced from 0.89 to 0.30.

TABLE 6

| Inhibition of human anti-p24 binding of 104-mer | | | | | | |
|---|---|---|---|---|---|---|
| | Patient Number | | | | | |
| Synthetic p24 ($\mu$g/ml) | | 50 | 5 | 0.5 | 0.05 | 0 |
| Mean Optical Density at 405nm. | 1 6 | 0.37 0.30 | 1.19 0.34 | 1.30 0.54 | 1.12 0.66 | 1.94 0.89 |

The data listed in Table 6 were obtained by incubating the 104-mer for 1 hour at room temperature with the individual serum diluted at 1 in 100. The mixture was then transferred to 104-mer coated wells and ELISA was performed as described above. The results are expressed in absorbance units. They represent the mean values of two experimental determinations.

The inhibition of binding of both sera to the 104-mer by the presence of synthetic 104-mer suggests that human antibodies which have been generated in the course of HIV infection and bind to native p24 recognise corresponding B epitope(s) on the synthetic 104-mer.

Monoclonal antibodies which had been raised against native HIV-1 p24 also bound to the 104-mer as shown in Figure 9. As these antibodies had previously been found to react with different B epitopes in p24, these results are suggestive that the 104-mer contains at least two distinct B epitopes which correspond to those of the viral p24 antigen. In Figure 9 the line ● - ● shows the results of the binding of monoclonal antibody IE and the line O--O the results of binding of monoclonal antibody CD expressed as mean O.D. 492 nm of duplicate assays at each dilution of the culture supernatant, SEM<10%.

Immunisation of BALB/c mice with the synthetic 104-mer gag polypeptide led to the production of specific IgG antibodies reactive to the 104-mer molecule in a solid-phase ELISA (Table 7). An IgG-specific second antibody was used in the ELISA. This indicates that T-cell help was generated.

No proliferative responses to synthetic 104-mer were found in healthy HIV carriers. This suggests that the p24 T cell of HIV carriers is compromised in some way.

Injection of the peptide into the mice also induced dose-dependent antigen-specific T-cell responses Table 7). T-cell responses (mean cpm ± standard deviations) to the peptide were determined for spleen cells of BALB/c (H-2$^d$) mice which were primed with 104-mer peptide emulsified in IFA one month earlier. Lymphocytes (5 x 10$^5$ cells per well) were restimulated with varying concentrations of the peptide in 96 flat-bottomed well plates (Costar, MA, USA) in a total volume of 200 $\mu$l of RPMI 1640 supplemented with 10% heat inactivated foetal calf serum (HIFCS), L-glutamine and 5 x 10$^{-5}$M 2-mercaptoethanol. Cultures were kept at 37°C in a humidified incubator equilibrated with 5% CO$_2$ for 3 days and pulsed (0.5 $\mu$Ci per well) for 4 hours with $^3$H-thymidine (Amersham International Ltd., U.K.) in the final incubation period. Radioactivity incorporation was determined by $\beta$-counting.

TABLE 7

| T and B cell responses to the 104-mer synthetic peptide. | | | | | | | | | |
|---|---|---|---|---|---|---|---|---|---|
| Antigen Dose ($\mu$g) | Peptide specific IgG at different serum dilutions (Mean optical density at 405 nm) | | | | Proliferation [($^3$H-Tdr uptake) cpm] to 104-mer[a] | | | | |
| | 1:10 | 1:10$^2$ | 1:10$^3$ | 1:10$^4$ | 50 ug/ml | 10 | 2 | 0.4 | - |
| 50 | 1.289 | 0.612 | 0.272 | 0.065 | 5764±481 | 5329±504 | 2324±363 | 692±74 | 472±56 |
| 10 | 0.699 | 0.335 | 0.077 | 0.002 | 2417±392 | 2894±417 | 1126±144 | 701±69 | 289±37 |
| 2 | 0.453 | 0.094 | 0.004 | 0.016 | 1421±163 | 1237±138 | 954±106 | 613±62 | 276±32 |
| -b | 0.003 | 0 | 0 | 0 | 431±51 | 376±42 | 281±24 | 307±44 | 427±59 |
| Notes: | | | | | | | | | |

a The results are expressed as mean cpm of triplicate cultures with standard deviations.
b Mice which received IFA alone are denoted -.

In vitro restimulation with the peptide of in vivo primed spleen cells resulted in substantial proliferation of the T lymphocytes. Optimal priming was observed with 50 $\mu$g of peptide within the range of the peptide concentrations tested. Significant (more than three-fold of background counts per minute) proliferation was seen after restimulation of the lymphocytes in the culture with 2, 10 and 50 $\mu$g of peptide. Optimal responses were observed at 10 to 50 $\mu$g ml$^{-1}$ of peptide. These data indicate that the 104-mer peptide contains one or more T-cell epitopes which act as a T-cell carrier for their preferred B epitopes and induce secondary antibody responses.

Example 3:

This Example describes the generation of 104-mer peptide specific long term murine T-cell lines.

Both recombinant and natural antigens have been used to generate long term T-cell lines and clones but the use of uncoupled peptides with unfused T-cell lines/clones has not been reported so far. Short peptides have been shown to induce short-term but not long-term propagation of T lymphocytes in the presence of exogenous interleukin-2 (IL-2) unless the T lymphocytes are immortalised by cell fusion to form T cell hybridomas. The ability of the 104-mer to prime T lymphocytes in vivo and induce proliferation of p24 specific memory T-cells in vitro promptedinvestigation whether the 104-mer could be used as an antigen to propagate the long-term growth of T-cells in the presence of exogenous IL-2. In order to generate 104-mer specific T-cell lines which contain lymphocyte clones that recognise all possible T-cell epitopes of the C-terminal half of p24, spleen cells from mice which had been primed (but not boosted) with the 104-mer were restimulated. The peptide-activated lymphocytes were then expanded and propagated by continuous culture in the presence of antigen, exogenous rat conA supernatant and irradiated autologous spleen cells as feeders. Thirty long term 104-mer specific T-cell lines were established and successfully kept in culture for over 5 months.

Example 4:

This Example describes mapping of the location of T-cell epitopes.

Mapping of the T-cell epitopes of an antigen requires the demonstration of all T-cells which are reactive to the possible T sites. Therefore 104-mer specific T-cell lines were chosen for use rather than clones for the mapping study, as isolation of clones from antigen-specific lines has been reported to result in in vitro selection by growth characteristics of the lymphocytes in addition to their peptide reactivities. One of the lines HIV p24/10 which demonstrated the best proliferation to the gag 104-mer was examined for reactivity against different peptides. The results in Table 8 clearly show that response of this T line is 104-mer specific.

TABLE 8

| Mapping of T-cell epitopes of 104-mer p24 by proliferation of line HIV p24/10 to short peptides | | | | |
|---|---|---|---|---|
| Peptide | Amino-acid sequence | $[3_H]$-Tdr uptake, counts per min (cpm) | | |
| | | 50 μg/ml | 10 μg/ml | 2 μg/ml |
| 104-mer | 273-377 | 6466±773 | 7411±997 | 3499±360 |
| PG2 | 273-290 | 486± 54 | 2511±296 | 3674±402 |
| PG-II | 291-305 | 1663±306 | 3265±517 | 1031±213 |
| PG3 | 312-326 | 83± 7 | 995± 93 | 2451±329 |
| PG4 | 330-346 | 1538± 82 | 3841±462 | 3540±371 |
| PG5 | 342-360 | 540±114 | 1505±432 | 594±210 |
| PG1 | 355-377 | 3623±331 | 4761±296 | 1149±246 |
| MRC p21 | 337-351 | 362± 53 | 1531±228 | 533± 76 |
| MRC p20 | 327-341 | 736±307 | 515±119 | 487±147 |
| MRC p19 | 317-331 | 617± 92 | 524± 81 | 290± 46 |
| MRC p18 | 307-321 | 427± 84 | 454±126 | 483±141 |
| MRC p17 | 297-311 | 397± 90 | 874± 52 | 428±112 |
| MRC p16 | 287-301 | 412±146 | 390± 82 | 357± 87 |
| MRC p15 | 277-291 | 1533±119 | 729±119 | 463± 89 |
| MRC p14 | 267-281 | 285±102 | 388± 61 | 336± 72 |
| MRC p13 | 257-271 | 300± 51 | 373± 60 | 335± 49 |
| MRC p12 | 247-261 | 265± 68 | 385± 60 | 224± 54 |
| MRC p11 | 237-251 | 253±112 | 361± 37 | 286±116 |
| tat | 1- 86 | 582± 57 | 597±105 | 367± 52 |
| - | | 342± 72 | | |
| conA 5μg/ml | | 22675±2880 | | |

Lymphocytes were harvested from culture. Viable lymphoblasts were recovered by centriguation over a Ficoll-Hypaque gradient. 1 x $10^6$ cells were then cultured for 5 days in RPMI 1640 supplemented with L-glutamine, 5 x $10^{-5}$ M 2-mercaptoethanol, 10% HIFCS and rat concanavalin A supernatant. Lymphocytes ere then washed three times in RPMI 1640 containing 5% FCS to remove the residual IL-2. 1 x $10^4$ cells were then cultured with varying concentrations of the peptide in the presence of 2 x $10^5$ irradiated (1500 R) autologous spleen cells as feeders. Cultures were incubated for 48 hours and pulsed overnight with $^3$H-thymidine. Radioactivity was measured by β-counting. No significant proliferation was observed with an unrelated long synthetic 86 residue peptide, tat-III (tat). The reactivity of this line was then tested with two sets of peptides to identify the predicted T epitopes. The first set (PG1, PG2, PG3, PG4, PG5 and P-II) contained peptides which were specially designed to include predicted T epitopes; the second set were 15-mer peptides (MRC p11-21) that overlap one another by 5 residues but were constructed without taking epitope predictions into consideration. Interestingly, proliferative responses were observed with all the predicted peptides (Table 8), and in addition with two peptides from the MRC set (MRC p15 and 21) which also cover predicted T epitopes. Optimal responses were obtained with 10 μg ml$^{-1}$ of antigen except for MRC p15 which required 50 μg ml$^{-1}$ and PG2 and PG3 which needed 2 μg/ml$^{-1}$. The highest level of proliferation was obtained with the predicted peptide PG1 (355-377) and the second most reactive peptide was PG2. Although peptide P-II (291-305) was recognised, two 15-mer peptides MRC p16 (287-301) and MRC p17 (297-311) which partially overlap the P-II site did not induce proliferation of HIV p24/10. Similarly PG3 peptide induced proliferation but the two overlapping 15-mers (MRC p18 and p19) did not. This suggests that MRC p16, MRC p17 and MRC p18 and p19, cut through the active P-II and PG3 sites respectively.

Example 5:

This Example demonstrates that both the correct and the reversed sequence of peptide 342-360 induce proliferation of the T-cell line.

A peptide was synthesised with the reverse sequence (i.e. from the C- to N-terminus of the peptide to that found in 342-360) because it was desired to investigate which residues are needed to induce the T-cell proliferation and to interact with MHC. The sequence contains a Rothbard-Taylor epitope and a predicted DeLisi and Berzofsky site. Both the correct and the reversed sequence induced proliferation of the 104-mer specific T-cell line.

Example 6:

This Example is provided to demonstrate that the T-cell line and T-cell clones recognise whole HIV-1.

In further assays both the T-cell line and T-cell clones to individual T epitopes of the 104-mer gave significant proliferation in the presence of whole live HIV-1. Two strains of HIV-1 were chosen for study because each yielded high titre virus. The supernatant of the cells was harvested and freeze-thawed several times to release the p24. Both viral lysates induce proliferation of the T-cell line and T-cell clones.

Example 7:

In this Example work is described which shows that the T-cell line is MHC-restricted.

Antigen presenting cells from $H_2^d$ mice but not from $H_2^q$ and $H_2^k$ mice induced proliferation with the synthetic 104-mer of the T-cell line. This indicates that the line is MHC-restricted. Furthermore inhibition of proliferation of the line to the 104-mer was observed with the anti-1-$A^d$/1-$E^d$ monoclonal antibody TIB 120 (see Table 9).

TABLE 9

| Inhibition of proliferation of line HIV p24/10 to 104-mer by monoclonal antibody TIB 120 (anti-1-$A^d$/1-$E^d$). | |
| --- | --- |
| Dilution of monoclonal antibody culture supernatant | 3H-Thymidine uptake |
| Neat | 176±46 |
| 1/10 | 201±67 |
| 1/100 | 433±180 |
| 1/1000 | 3484±470 |

Example 8:

The cell line is a T helper phenotype since phenotypic analysis of the line HIV p24/10 revealed that 92% of the lymphocytes are Thy 1.2 antigen positive which indicates that they are T-cells. The majority (74%) of these cells are L3T4 phenotype which is the T helper subset. Only 2% were Lyt 2 antigen positive or the suppressor/cytotoxic phenotype.

Example 9:

Upon investigation of the T-cell responses to synthetic 104-mer p24 in HIV-infected carriers, it was found that the synthetic 104 mer failed to induce T-cell proliferation in lymphocytes which were isolated from the blood of 6 healthy HIV carriers. Even individuals with normal CD4 counts and good anti-104-mer antibody levels did not show anti-p24 T-cell responses.

Example 10:

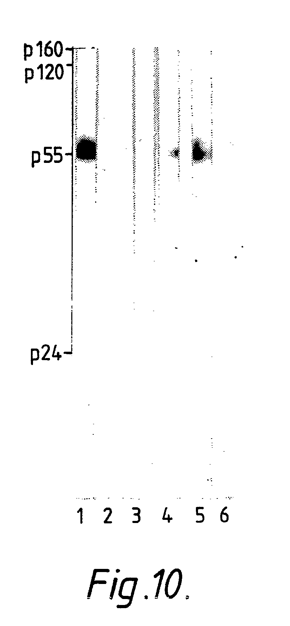

Whilst priming followed by boosting of mice with the envelope peptide (775-799) resulted in no antibody formation against HIV-1 proteins, immunisation with the core T epitope containing peptides yielded antibody to the p24 precursor, p55. In Figure 10 is shown the binding of mouse sera from T epitope immunised mice to the viral core precursor p55 in immunoblots. In Figure 10 lane 1 shows reactivity of murine BALB/C serum from PG2 immunised mouse to HIV-1 viral proteins. Lanes 2 to 6 are similar except that mice were injected with P-II, PG3, PG4, PG5 and PG1 respectively. Whilst all T epitope containing peptides yielded antibody to p55, PG2 and PG5 gave particularly intense staining. The antisera from mice immunised with these six short peptides also reacted with HIV-1 isolate 82 and with 104-mer in ELISAs (data not shown).

Example 11:

By a process analogous to that described in Example 1 there is produced the 94-mer polypeptide of the formaule (I) wherein X represents the carboxylic acid group on the L residue 367. This is found to be a valuable tool in investigating the presence of T-cell epitopes in the gag p24 polypeptide.

**Claims**

1. Synthetic polypeptides of the following formula:

     280        290        300        310        320

LNKIVRMYSPTSILDIRQGPKEPFRDYVDRFYKTLRAEQASQEVKNWMTETL

     330        340        350        360

LVQNANPDAKYILKALGPAATLEEMMTAAQGVGGPGHKARVL-X     (I)

where X represents a the carboxylic acid group of the L residue 367 or the polypeptide sequence:

     370

AEAMSQVTNS.

2. Polypeptides of the formula (I) set out in claim 1 in which residues 334 and 354 are changed from a residue of alanine to the residue of another neutral amino acid or to a residue of cysteine.

3. Synthetic polypeptides which consist of or which contain at least 5 consecutive residues found in the following polypeptide sequence:
LEEMMTAAQGVG    (II).

4. Synthetic polypeptides which consist of or which contain the polypeptide sequence:

LEEMMTAAQGVG    (II).

5. Synthetic polypeptides which consist of at least 75 amino acid residues and which contain the polypeptide sequence:

LEEMMTAAQGVG    (II).

6. Synthetic polypeptides which consist of or which contain at least 5 consecutive residues found in the polypeptide sequence:

LEEMMTAΦQGVG    (III),

where Φ represents an alanine residue or a residue of another neutral amino acid or a residue of cysteine.

7. Polypeptides of the following formula:

θ-LEEMMTAΦQGVG-δ    (IV),

where θ represents a polypeptide sequence containing up to 80 amino acid residues, Φ represents an alanine residue, a residue of a neutral amino acid other than alanine, or a residue of cysteine, and δ represents a polypeptide sequence containing up to 25 amino acid residues.

8. Polypeptides according to claim 7, in which θ represents a polypeptide sequence containing up to 73 amino acid residues and δ represents an amino acid sequence containing up to 19 amino acid residues.

9. Polypeptides according to claim 7 or claim 8, in which θ and δ represent portions of the p24 peptide found in a human immunodeficiency virus of the type 1 (HIV-1) or type 2 (HIV-2) or in the virus SIV$_{MAC}$.

10. T-cell epitopes being polypeptide fragments included within a polypeptide according to any one of claims 1 to 9.

11. Polypeptides of one of the following formulae:

(i)

360       370

QGVGGPGHKARVLAEAMSQVTNS    (V)

(ii)

280       290

LNKIVRMYSPTSILDIR    (VI)

(iii)

320

QASQEVKNWMTETLL    (VII)

(iv)

330       340

ANPDAKYILKALGPAAT    (VIII)

(v)

350       360

GPAATLEEMMTAAQGVGGP    (IX)

(vi)

300

QGPKEPFRDYVDRFY    (X)

(vii)

PGGVGQAATMMEELTAAPG    (XI).

12. Antibodies to a polypeptide according to any one of claims 1 to 11.

13. A vaccine comprising a polypeptide according to any one of claims 1 to 11.

14. A vaccine according to claim 13, which further contains at least one other polypeptide having a protective effect against a human immunodeficiency virus of type 1 (HIV-1) or type 2 (HIV-2) or against the virus SIV$_{MAC}$.

15. A polypeptide according to any one of claims 1 to 11 for use as an immunogen.

16. A diagnostic agent comprising a polypeptide according to any one of claims 1 to 11 or an antibody according to claim 12.

EP 0 356 007 A2

Fig.1.

Fig.2.

EP 0 356 007 A2

Fig.3.

Fig .4.

Fig.5.

CONTROL    104 −MER
            IMMUNIZED MOUSE

d

160,000
120,000

65,000
55,000
41−43,000

32,000

24,000

18,000

*Fig.6.*

KDa

160
120

55

24

1  2  3  4  5  6  7    *Fig.7.*

Fig.8.

Fig.9.

Fig.10.